# FASCICULE DE BREVET EUROPEEN

(11) **EP 1 019 017 B1**
(45) Date de publication et mention de la délivrance du brevet: **30.10.2002**
(21) Numéro de dépôt: 98912574.5
(22) Date de dépôt: 02.03.1998
(51) Int. Cl.: A61K 7/48

(54) **ASSOCIATION DE DERIVES DU PHENOL ET D'UN EXTRAIT D'IRIDACEES POUR LA DEPIGMENTATION DE LA PEAU OU DE SES PHANERES ET COMPOSITION LA COMPRENANT**
KOMBINATION AUS PHENOLDERIVATEN UND EINEM EXTRAKT VON IRIDACEEN ZUR DEPIGMENTIERUNG DER HAUT ODER IHRER ANHANGSGEBILDE UND DIESE KOMBINATION ENTHALTENDE ZUSAMMENSETZUNG
ASSOCIATION OF PHENOL DERIVATIVES WITH EXTRACT OF IRIDACEAE FAMILY FOR LIGHTENING THE SKIN AND SUPERFICIAL BODY GROWTH AND COMPOSITION CONTAINING SAME

(30) Priorité: 03.03.1997 FR 9702504
(43) Date de publication de la demande: 19.07.2000
(73) Titulaire: L'OREAL, 75008 Paris (FR)
(72) Inventeur: BRETON, Lionel, F-78000 Versailles (FR)
(74) Mandataire: Tezier Herman, Béatrice
(86) Numéro de dépôt international: FR9800402
(87) Numéro de publication internationale: WO98038978

(56) Documents cités:
- EP-A- 0 570 230
- WO-A-96/24327
- FR-A- 2 732 594
- DATABASE WPI Week 9705 Derwent Publications Ltd., London, GB; AN 97-048238 XP002045139 "Use of Eleutherol as external dermal agent for skin whitening - useful in inhibiting melanin formation and tyrosinase activity" & JP 08 301 758 A (SHISEIDO) , 19 novembre 1996
- DATABASE WPI Week 8543 Derwent Publications Ltd., London, GB; AN 85-266685 XP002045140 "Mugwort-based bathing compsn. prepn. - using an alcohol as binder and a specified dispersing agent" & JP 60 178 811 A (MATSUDA IYAKUHIN) , 12 septembre 1985
- DATABASE WPI Week 9514 Derwent Publications Ltd., London, GB; AN 95-101793 XP002045141 "External use agent for removing freckles and spots from skin - comprises kojic acid (deriv.) and one or more plant extracts, e.g. from iceland moss, orris root, oak bark, gardenia, mallow, avens, citrus unshiu peel, grape, cornflower, etc." & JP 07 025 762 A (SANSWI SEIYAKU) , 27 janvier 1995
- DATABASE WPI Week 9715 Derwent Publications Ltd., London, GB; AN 97-161403 XP002045172 "An external compsn for fair skin for e g sunburn treatment - comprises solvent extract of Iridaceae family" & JP 09 030 954 A (SHISEIDO) , 4 février 1997
- DATABASE WPI Week 9806 Derwent Publications Ltd., London, GB; AN 98-059103 XP002066707 "Belamcanda chinensis L. extracts, cell activating agent contg. alpha-hydroxy acid, and their usage - used for bathing compositions or skin external compositions" & JP 09 301 883 A (ICHIMARU PHARCOS) , 25 novembre 1997

## Description

La présente invention se rapporte à l'association de dérivés du phénol et d'extraits d'lridacées, les compositions cosmétiques ou dermatologiques comprenant une telle association et leur utilisation par application topique sur la peau et/ou ses phanères, dans le but de blanchir la peau et/ou ses phanères.

La couleur de la peau humaine est fonction de différents facteurs et notamment des saisons de l'année, de la race et du sexe, et elle est principalement déterminée par la nature et la concentration de mélanine produite par les mélanocytes. Les mélanocytes sont les cellules spécialisées qui par l'intermédiaire d'organelles particuliers, les mélanosomes, synthétisent la mélanine. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

De la même manière, la couleur des poils et des cheveux est due à la mélanine, lorsque les poils ou les cheveux sont foncés, certaines personnes désirent voir ceux-ci plus claires. Ceci est particulièrement intéressant pour les poils qui sont moins visibles lorsqu'ils sont clairs que lorsqu'ils sont foncés.

Le mécanisme de formation de la pigmentation de la peau, des poils et des cheveux, c'est-à-dire de la formation de la mélanine est particulièrement complexe et fait intervenir schématiquement les principales étapes suivantes :
Tyrosine ---> Dopa ---> Dopaquinone ---> Dopachrome ---> Mélanine

La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase. Cette tyrosinase n'agit que lorsqu'elle est à l'état de maturation sous l'action de certains facteurs biologiques.

Une substance est reconnue comme dépigmentante si elle agit directement sur la vitalité des mélanocytes épidermiques où se déroule la mélanogénèse et/ou si elle interfère avec une des étapes de la biosynthèse de la mélanine soit en inhibant une des enzymes impliquées dans la mélanogénèse soit en s'intercalant comme analogue structural d'un des composés chimiques de la chaîne de synthèse de la mélanine, chaîne qui peut alors être bloquée et ainsi assurer la dépigmentation.

Les substances les plus utilisées en tant que dépigmentants sont plus particulièrement l'hydroquinone et ses dérivés, en particulier ses éthers tels que le monométhyléther et le monoéthyléther d'hydroquinone. Ces composés, bien qu'ils présentent une efficacité certaine, ne sont malheureusement pas exempts d'effets secondaires du fait de leur toxicité, ce qui peut rendre leur emploi délicat, voire dangereux. Cette toxicité provient de ce qu'ils interviennent sur des mécanismes fondamentaux de la mélanogénèse en tuant des cellules qui risquent alors de perturber leur environnement biologique et qui par conséquent obligent la peau à les évacuer en produisant des toxines.

Ainsi, l'hydroquinone est un composé particulièrement irritant et cytotoxique pour le mélanocyte, dont le remplacement, total ou partiel a été envisagé par de nombreux auteurs.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne, telles que les taches pigmentaires séniles dites lentigo actiniques, les hyperpigmentations ou dépigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir à une cicatrice donnant à la peau un aspect plus blanc et les leucodermies), à défaut de pouvoir repigmenter la peau lésée, on achève de dépigmenter les zones de peau normale résiduelle pour donner à l'ensemble de la peau une teinte blanche homogène.

Dans ce sens, les dérivés de l'acide salicylique et le paracétamol sont des inhibiteurs de la tyrosinase et, à ce titre, sont décrits comme des dépigmentants.

On utilise également couramment, comme inhibiteur de la tyrosinase, l'acide kojique qui complexe le cuivre présent dans le site actif de cette enzyme. Malheureusement, ce composé peut provoquer des réactions d'allergie ("Contact allergy to kojic acid in skin care products", Nakagawa M. et al., in Contact Dermatitis, Jan. 95, Vol 42 (1), pp.9-13). Ce composé est également instable en solution, ce qui complique quelque peu la fabrication de la composition.

JP 9301883 divulgue la combinaison d'extrait d'Iridacé avec une acide α-hydroxylique comme agent pour activer las cellules. EP-A-570 230 divulgue certaines dérivés du phénol avec une activité dépigmentante.

Un des buts de la présente invention est donc de proposer de nouveaux produits présentant une activité dépigmentante particulièrement efficace, tout en ne présentant les inconvénients décrits ci-dessus, tels que la toxicité et le pouvoir allergisant.

La demanderesse a trouvé de manière inattendue que certains dérivés du phénol en association avec des extraits d'lridacées présentaient un synergie vis-à-vis de la propriété dépigmentante de la peau et/ou de ses phanères.

En effet, sans vouloir se lier à une quelconque théorie de l'invention, il semble que, du fait de la propriété tout à fait inattendu des extraits d'lridacées qui est d'antagoniser les effets de neuropeptides cutanés, tel que le CGRP, ces extraits permettent d'inhiber l'activation, due au CGRP, de la synthèse de la mélanine via une action positive sur la sécrétion d'AMPc intracellulaire dans les mélanocytes. Ainsi, l'activité dépigmentante des extraits d'lridacées associée à celle de certains dérivés du phénol permet de blanchir ou de dépigmenter la peau ou les phanères de manière tout à fait remarquable.

L'acide salicylique et ses dérivés sont connus comme des agents kératolytiques qui peuvent donc réduire à doses élevées (supérieures à 3% en poids) les taches pigmentaires en éliminant les cellules mortes en surface de la peau et notamment celles de ces taches. Mais, ces composés à plus faibles doses (inférieures à 3%) en poids peuvent également inhiber l'activité de la tyrosinase en ayant pour cible les mélanocytes, et permettre ainsi une réduction de la production de mélanine, tout en ne présentant pas une activité inflammatoire comme lorsqu'ils sont utilisés à fortes doses.

Aussi, la présente invention a pour objet un produit de combinaison constitué par l'association d'extrait d'au moins une Iridacée et d'au moins un dérivé du phénol de formule (I): 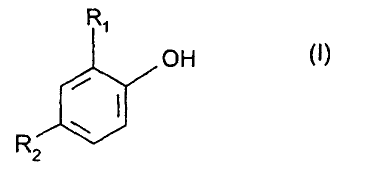 dans laquelle :
R₁ représente un atome d'hydrogène ou un radical acide,
R₂ représente un atome ou un radical choisi parmi l'atome d'hydrogène, un radical de formule -NHCOCH₃ et un radical de formule -COR₃, dans laquelle R₃ représente une chaîne aliphatique saturée, linéaire, ramifiée ou cyclisée, une chaîne insaturée portant une ou plusieurs doubles liaisons conjuguées ou non, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone,
avec la condition que lorsque R₁ représente un atome d'hydrogène, R₂ ne représente pas un atome d'hydrogène,
et ses sels ou esters cosmétiquement ou pharmaceutiquement acceptable.

De préférence, lorsque R₁ représente un atome d'hydrogène, R₂ représente le radical -NHCOCH₃.

De préférence, lorsque R₁ représente un radical acide, R₂ représente le radical de formule -COR₃ ou un atome d'hydrogène.

De préférence, le radical R₃ comporte au moins 4 atomes de carbone. Il est par exemple formé d'un radical alkyle linéaire saturé ayant de 4 à 11 atomes de carbone.

Ainsi, de manière préférentielle, le dérivé du phénol est choisi parmi le paracétamol (dénommé le N-(4-hydroxyphenyl)acétamide ou encore acétaminophène), l'acide salicylique et les dérivés de l'acide salicylique.

Lorsque le dérivé du phénol est un dérivé de l'acide salicylique, il est préférentiellement choisi parmi les acides n-octanoyl-5-salicylique, n-décanoyl-5-salicylique, n-dodécanoyl-5-salicylique.

II peut s'agir également des sels de ces acides, et en particulier des sels obtenus par salification avec une base. Comme base susceptible de salifier les dérivés de l'acide salicylique selon l'invention, on peut citer les bases minérales comme les hydroxydes de métaux alcalins (hydroxydes de sodium et de potassium) ou les hydroxydes d'ammonium ou mieux encore les bases organiques.

De préférence, on utilise des bases amphotères pour la salification des dérivés de l'acide salicylique, c'est-à-dire des bases ayant à la fois des groupements fonctionnels anioniques et cationiques. Les bases amphotères peuvent être des amines organiques primaires, secondaires, tertiaires ou cycliques et plus spécialement des acides aminés. A titre d'exemple de bases amphotères, on peut citer la glycine, la lysine, l'arginine, la taurine, l'histidine, l'alanine, la valine, la cystéïne, la trihydroxy-méthylaminométhane (TRISTA), la triéthanolamine. Ces bases sont utilisées en quantités suffisantes pour amener le pH de l'émulsion entre 5 et 7 et donc proche de celui de la peau. Il s'ensuit une grande compatibilité de l'émulsion de l'invention vis-à-vis de la peau.

L'extrait d'au moins une Iridacée est un extrait dudit végétal obtenu par culture *in vitro* ou *in vivo.*

Le terme "extrait d'au moins une Iridacée" doit s'entendre comme "extrait de cellules d'lridacées" et donc comme "extrait de cellules d'au moins un végétal de la famille des Iridacées".

L'extrait d'au moins un Iridacée peut être un extrait préparé à partir de tout matériel végétal issu de la famille des Iridacées, ledit matériel ayant été obtenu par culture *in vitro* ou *in vivo*.

La pression de sélection imposée par les conditions physico-chimiques lors de la croissance des cellules végétales *in vitro* permet d'obtenir un matériel végétal standardisé et disponible tout au long de l'année contrairement aux plantes obtenu par culture *in vivo*. Ainsi, on préfère utiliser un extrait d'lridacées provenant d'une culture *in vitro,* ce qui n'exclut pas que l'iridacée cultivé au départ *in vitro* puisse être ensuite cultivé *in vivo.*

Par culture *in vitro*, on entend l'ensemble des techniques connues de l'homme du métier qui permet de manière artificielle l'obtention d'un végétal ou d'une partie d'un végétal.
Ainsi, par exemple selon l'invention l'extrait peut être un extrait d'organe, voire de cellules d'organe, d'au moins une Iridacée obtenu par culture *in vitro* (racines, tige, feuille) ou encore un extrait de cellules indifférenciées d'au moins une Iridacée.

De préférence on utilise un extrait obtenu à partir de cellules indifférenciées obtenues par culture *in vitro.*

Par cellules végétales indifférenciées, on entend toute cellule végétale ne présentant aucun des caractères d'une spécialisation particulière et capable de vivre par elle-même et non en dépendance avec d'autres cellules. Ces cellules végétales indifférenciées sont éventuellement aptes, sous l'effet d'une induction, à toute différenciation conforme à leur génome.
Selon la méthode de culture choisie, et en particulier selon le milieu de culture choisi, il est possible d'obtenir à partir d'un même explant des cellules végétales indifférenciées présentant des caractères différents.

La famille des Iridacées (ou Iridées) compte environ 750 espèces.
Les plantes de la famille des Iridacées sont surtout utilisées pour leur propriétés aromatiques et ornementales.
Parmi les genres d'Iridacées utilisables selon l'invention, on peut citer à titre d'exemple les genres Romulea, Crocus, Iris, Gladiolus, Sisyrinchium ou encore Hermodactylus.
Comme matériel végétal utilisable on peut citer celui provenant d'*Iris germanica,* d'*Iris florentina,* d'*Iris pallida,* de *Crocus versicolor,* de *Romulea bulbucodium* ou encore de *Gladiolus communis*.

Plus particulièrement selon l'invention on utilise du matériel végétal issu du genre Iris, plus particulièrement d*'Iris pallida*.

Toute méthode d'extraction connue de l'homme du métier peut être utilisée selon l'invention.
On peut en particulier citer les extraits alcooliques, notamment éthanoliques, les extraits hydroalcooliques.

On peut également utiliser un extrait préparé par la méthode décrite dans la demande de brevet français n° 95-02379.
Ainsi, dans une première étape on broie le matériel végétal dans une solution aqueuse à froid, dans une deuxième étape les particules en suspension sont éliminées de la solution aqueuse issue de la première étape, et dans une troisième étape on stérilise la solution aqueuse issue de la deuxième étape. Cette solution aqueuse peut correspondre à l'extrait.
D'autre part, la première étape peut avantageusement être remplacée par une opération de congélation simple des tissus végétaux (par exemple à -20°C), suivie d'une extraction aqueuse reprenant les deuxièmes et troisième étapes ci-dessus décrites.
Un exemple de préparation d'extrait utilisable selon l'invention est donné par ailleurs dans les exemples.

Les extraits obtenus selon les procédés précédemment décrits peuvent être utilisés tels que ou bien être condensés. Ils peuvent être également utilisés sous forme sèche, après retrait du solvant, notamment par lyophilisation.

La présente invention concerne également une composition topique, cosmétique ou dermatologique, comprenant l'association définie ci-dessus, comme agent de blanchiment ou dépigmentant, et un véhicule approprié cosmétiquement ou pharmaceutiquement acceptable.

Elle concerne également l'utilisation de l'association de la présente invention pour blanchir et/ou dépigmenter la peau ou ses phanères dans ou pour la fabrication d'une composition cosmétique ou pharmaceutique.

Elle a trait enfin à un procédé cosmétique pour blanchir ou dépigmenter la peau ou ses phanères, caractérisé en ce que l'on applique sur la peau ou ses phanères une composition cosmétique comprenant l'association définie ci-dessus et un véhicule approprié cosmétiquement acceptable.

Les phanères sont de préférence les poils ou les cheveux.

Cette association s'avère très efficace pour diminuer, voire supprimer les taches pigmentaires de la peau.

La composition selon l'invention est appropriée pour une utilisation topique et contient donc un milieu cosmétiquement ou dermatologiquement acceptable, c'est-à-dire compatible avec la peau ou ses phanères, plus particulièrement les poils ou les cheveux.

Les quantités des dérivés du phénol et de l'extrait d'au moins une Iridacée peuvent varier dans une large mesure et dépendent notamment de l'activité dépigmentante désirée.

Ainsi, pour donner un ordre de grandeur, les dérivés du phénol sont utilisés, selon la présente invention, généralement en quantité comprise entre 0,001 et 10 %, de préférence entre 0,1 et 5%, en poids par rapport au poids total de la composition, encore plus préférentiellement de 0,2 et 3 % en poids.

Pour donner un ordre de grandeur, l'extrait d'au moins un Iridacée est pour sa part utilisé de préférence en quantité sèche comprise entre 0,0001 et 20 % en poids par rapport au poids total de la composition, plus préférentiellement comprise entre 0,001 et 10 % en poids, et encore plus préférentiellement comprise entre 0,001 et 5 % en poids.

L'application d'une composition contenant l'association d'extrait d'lridacées et d'un dérivé du phénol selon l'invention permet d'obtenir une nette diminution, voire une disparition complète, de la formation de taches, même en employant des quantités de dérivés du phénol, plus particulièrement de l'acide salycique ou de ses dérivés; inférieures à celles préconisées dans l'état de la technique.

Les compositions contenant l'association selon l'invention peuvent présenter toutes les formes galéniques normalement utilisées pour une application topique, par exemple sous forme de solutions aqueuses, hydroalcooliques ou huileuses, de dispersions du type lotion ou sérum, de gels aqueux, anhydres ou huileux, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore de microémulsions, de microcapsules, de microparticules ou de dispersions vésiculaires de type ionique et/ou non ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau ou sur ses phanères sous forme d'aérosol. Elle peut également se présenter sous forme solide, et par exemple sous forme de stick. Elle peut être utilisée comme produit de soin et/ou comme produit de maquillage. Elle peut également être sous une forme de shampooings ou après-shampooings.

Ces compositions sont préparées selon les méthodes usuelles.

De façon connue, les compositions cosmétiques ou dermatologiques de l'invention peuvent contenir des adjuvants habituels dans le domaine cosmétique ou dermatologique, tels que les émulsionnants, les gélifiants hydrophiles ou lipophiles, les actifs hydrophiles ou lipophiles, les conservateurs, les antioxydants, les parfums, les charges, les filtres et les matières colorantes. Les quantités de ces différents adjuvants sont celles classiquement utilisées dans les domaines cosmétique et/ou dermatologique, et par exemple de 0,01 % à 20 % du poids total de la composition. Ces adjuvants, selon leur nature, peuvent être introduits dans la phase grasse, dans la phase aqueuse et/ou dans les vésicules lipidiques.

Comme émulsionnants, on peut utiliser des émulsionnants eau-dans huile (E/H) ou huile-dans-eau (H/E) selon l'émulsion finale souhaitée. Comme émulsionnants, on peut citer par exemple le stéarate de PEG-20, le stéarate de PEG-100, le Polysorbate 60 (Tween 60 vendu par la société ICI, le stéarate de sorbitan (Span 60 vendu par la société ICI) et le PPG-3 myristyl éther.

Le taux d'émulsionnant peut aller de 0,1 % à 15 % en poids, et de préférence de 0,5 % à 5 % en poids, par rapport au poids total de la composition.

On peut ajouter à la composition selon l'invention des coémulsionnarits, par exemple en une quantité allant de 0,05 % à 10 % en poids par rapport au poids total de la composition. Comme coémulsionnant, on peut citer le stéarate de glycérol.

Dans les dispersions de vésicules lipidiques, l'émulsionnant peut être constitué par des vésicules de lipides ioniques et/ou non ioniques.

Comme huiles utilisables dans l'invention, on peut citer les huiles minérales, les huiles végétales (huile de tournesol, huile d'amande d'abricot, huile de karité), les animales (lanoline), les huiles de synthèse, les huiles siliconées (cyclométhicone) et les huiles fluorées (perfluoropolyéthers). On peut ajouter à ces huiles des alcools gras (alcool stéarylique), des acides gras (acide stéarique) et des cires.

Comme gélifiants hydrophiles, on peut citer les polymères carboxyvinyliques, les polyacrylates ou polyméthacrylates de glycéryle, les polyacrylamides, les gommes naturelles (xanthane) et les argiles, et, comme gélifiants lipophiles, on peut citer les argiles modifiées comme les bentones, les sels métalliques d'acides gras comme les stéarates d'aluminium et la silice hydrophobe.

Comme actifs hydrophiles, on peut utiliser par exemple les protéines ou les hydrolysats de protéine, les acides aminés, les polyols, en particulier la glycérine ou le sorbitol, l'urée, l'allantoïne, les sucres et leurs dérivés, l'acide glycyrrhétinique.

Comme actifs lipophiles, on peut citer le tocophérol (vitamine E) et ses dérivés, les acides gras essentiels, les céramides, les huiles essentielles.

On peut aussi utiliser dans ces compositions des filtres UV à propriété lipophile ou hydrophile, les oxydes de titane et de zinc, éventuellement sous forme de nano-oxydes, notamment en vue d'obtenir des compositions assurant également une bonne protection contre les UV.

Bien entendu, l'homme du métier veille à ne pas ajouter des composés contrevenant aux effet attendus, et plus particulièrement contrevenant à l'effet dépigmentant de la composition.

Ces compositions constituent notamment des crèmes de protection, de traitement ou de soin pour le visage, pour les mains ou pour le corps, des laits corporels de protection ou de soin, des lotions, gels ou mousses pour le soin ou le traitement de la peau, des lotions de nettoyage ou de désinfection, des compositions pour le bain, des fonds de teint et des crèmes teintées. Dans ces derniers cas, la composition contient des pigments.

Les exemples de compositions ci-après permettent d'illustrer l'invention, sans chercher à en limiter la portée. Sauf mention contarires, les pourcentages sont donnés en poids.

### Exemple 1

### Préparation d'un extrait d'Iris pallida :

Des cellules indifférenciées d'*Iris pallida* obtenu par culture *in vitro* en conditions axéniques sont récupérées après culture en Erlenmeyer ou en fermenteur par filtration sur tamis de 50µm. A 55 g de matière fraîche ainsi obtenue, on ajoute 27,5 ml d'eau déminéralisée. L'ensemble est broyé (Potter, Ultra Turrax...) au Turrax à 24000 T/min durant 1 minute à 4°C (bain de glace). Le broyât est centrifugé 15 à 10000 G à 4°C. Le surnageant est filtré à 0,22 µm (filtration stérilisante).
L'extrait ainsi préparé est conservé à 4°C. Il renferme environ 15 g de matière sèche par litre. L'extrait obtenu peut être lyophilisé.
Si le matériel végétal est de la plante entière, on ramène la matière fraîche à traiter en fonction du poids sec pour se mettre dans les mêmes conditions d'extraction que pour l'*in vitro.* Les différentes parties de la plante sont prélevées en fonction du poids relatif de chaque partie de celle-ci. Le traitement à froid permet dé geler les activités enzymatiques, la filtration stérilisante évite la dégradation des actifs par les micro-organismes de l'environnement. Enfin, le véhicule eau facilite les formulations cosmétiques ou pharmaceutiques.

### Exemple 2

### Lotion dépigmentante

| **Ingrédients** | **%** |
|---|---|
| Extrait de l'exemple 1 lyophilisé | 2,00 |
| Acide capryloyl salicylique | 2,00 |
| Ethanol | 48,00 |
| Eau | 9,40 |
| Polyéthylène glycol (PEG-8) | 38,50 |
| Conservateur | 0,10 |

### Exemple 3

### Gel dépigmentant

| **Ingrédients** | **%** |
|---|---|
| Extrait de l'exemple 1 lyophilisé | 2,00 |
| Acide capryloyl salicylique | 2,00 |
| Ethanol | 45,50 |
| Eau | 9,00 |
| Polyéthylène glycol (PEG-8) | 36,40 |
| Conservateur | 0,10 |
| Gélifiant (Polyacrylamide + isoparaffine en C₁₃-C₁₄ + laureth-7) | 5,00 |

## Revendications

1. Produit de combinaison constitué par l'association d'extrait d'au moins une Iridacée et d'au moins un dérivé du phénol de formule (I): 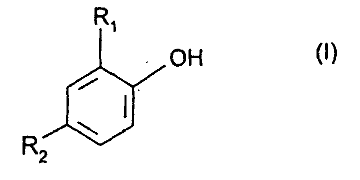 dans laquelle:
R₁ représente un atome d'hydrogène ou un radical acide,
R₂ représente un atome ou un radical choisi parmi l'atome d'hydrogène, un radical de formule -NHCOCH₃ et un radical de formule -COR₃, dans laquelle R₃ représente une chaîne aliphatique saturée, linéaire, ramifiée ou cyclisée, une chaîne insaturée portant une ou plusieurs doubles liaisons conjuguées ou non, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone.
avec la condition que lorsque R₁ représente un atome d'hydrogène, R₂ ne représente pas un atome d'hydrogène,
et ses sels ou esters cosmétiquement ou pharmaceutiquement acceptable.

2. Produit de combinaison selon la revendication 1, **caractérisé en ce que** le dérivé du phénol présente une formule (I) dans laquelle R₁ représente un atome d'hydrogène et R₂ représente le radical -NHCOCH₃.

3. Produit de combinaison selon la revendication 1, **caractérisé en ce que** le dérivé du phénol présente une formule (I) dans laquelle R₁ représente un radical acide, R₂ représente le radical de formule -COR₃ ou un atome d'hydrogène.

4. Produit de combinaison selon la revendication 1, **caractérisé en ce que** le dérivé du phénol est choisi parmi le paracétamol, l'acide salicylique et les dérivés de l'acide salicylique.

5. Produit selon la revendication 4, **caractérisée en ce que** les dérivés de l'acide salicylique sont choisis parmi les acides n-octanoyl-5-salicylique, n-décanoyl-5-salicylique, n-dodécanoyl-5-salicylique.

6. Produit selon l'une quelconque des revendications précédentes, **caractérisé en ce que** l'Iridacée de l'extrait provient d'une Iridacée d'un genre choisi parmi Romulea, Crocus, Iris, Gladiolus, Sisyrinchium et Hermodactylus.

7. Produit selon la revendication précédente, **caractérisé en ce que** ledit extrait est un extrait de matériel végétal provenant d'Iris.

8. Produit selon la revendication précédente, **caractérisée en ce que** ledit extrait est un extrait de matériel végétal provenant *d'Iris pallida*.

9. Composition topique, cosmétique ou dermatologique, comprenant l'association telle que définie dans Tune quelconque des revendications précédentes et un véhicule approprié cosmétiquement ou pharmaceutiquement acceptable.

10. Composition selon la revendication précédente, **caractérisée en ce que** les dérivés du phénol sont utilisés en quantité comprise entre 0,001 et 10 %, de préférence entre 0,1 et 5%, en poids par rapport au poids total de la composition.

11. Composition selon l'une des revendications 9 ou 10, **caractérisée en ce que** l'extrait d'au moins un Iridacée est utilisé en quantité sèche comprise entre 0,0001 et 20 % en poids par rapport au poids total de la composition, plus préférentiellement comprise entre 0,001 et 10 % en poids, et encore plus préférentiellement comprise entre 0,001 et 5 % en poids.

12. Utilisation du produit défini selon l'une quelconque des revendications 1 à 8 pour blanchir et/ou dépigmenter la peau ou ses phanères dans une composition cosmétique ou pour la fabrication d'une composition pharmaceutique.

13. Procédé cosmétique pour blanchir ou dépigmenter la peau ou ses phanères, **caractérisé en ce que** l'on applique sur la peau ou ses phanères une composition cosmétique définie selon l'une quelconque des revendications 9 à 11.

## Patentansprüche

1. Kombinationsprodukt, das aus der Kombination eines Extrakts mindestens einer Iridacea und mindestens eines Phenolderivats der Formel (I) 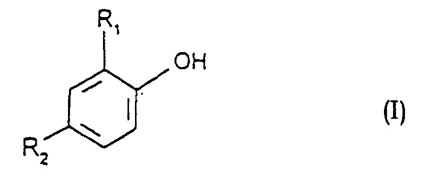 besteht, in der bedeuten:
R₁ ein Wasserstoffatom oder einen Säurerest,
R₂ ein Atom oder einen Rest, das/der unter Wasserstoff, der Gruppe der Formel -NHCOCH₃ und den Gruppen der Formel -COR₃ ausgewählt ist, worin R₃ bedeutet: eine geradkettige, verzweigte oder ringförmige gesättigte aliphatische Kette, eine ungesättigte Kette, die eine oder mehrere konjugierte oder nicht konjugierte Doppelbindungen enthält, wobei diese Ketten 2 bis 22 Kohlenstoffatome aufweisen können und substituiert sein können mit mindestens einem Substituenten, der unter den Halogenatomen, der Trifluormethylgruppe, Hydroxygruppen in freier Form oder verestert mit einer Säure, die 1 bis 6 Kohlenstoffatome aufweist, ausgewählt ist, oder mit einer Carboxygruppe in freier Form oder verestert mit einem niederen Alkohol, der 1 bis 6 Kohlenstoffatome aufweist,
mit der Maßgabe, daß R₂ kein Wasserstoffatom darstellt, wenn R₁ ein Wasserstoffatom bedeutet,
und seine kosmetisch oder pharmazeutisch akzeptalen Salze.

2. Kombinationsprodukt nach Anspruch 1, **dadurch gekennzeichnet, daß** das Phenolderivat einer Verbindung der Formel (I) entspricht, in der R₁ ein Wasserstoffatom und R₂ die Gruppe -NHCOCH₃ bedeutet.

3. Kombinationsprodukt nach Anspruch 1, **dadurch gekennzeichnet, daß** das Phenolderivat einer. Verbindung der Formel (I) entspricht, in der R₁ eine Säuregruppe und R₂ eine Gruppe der Formel -COR₃ oder ein Wasserstoffatom darstellt.

4. Kombinationsprodukt nach Anspruch 1, **dadurch gekennzeichnet, daß** das Phenolderivat unter Paracetamol, Salicylsäure und den Salicylsäurederivaten ausgewählt ist.

5. Produkt nach Anspruch 4, **dadurch gekennzeichnet, daß** die Salicylsäurederivate unter 5-(n-Octanoyl)-salicylsäure, 5-(n-Decanoyl)-salicylsäure, 5-(n-Dodecanoyl)-salicylsäure ausgewählt sind.

6. Produkt nach einem der vorhergehenden Ansprüchen, **dadurch gekennzeichnet, daß** der Iridacea-Extrakt von einer Iridacea einer Gattung stammt, die unter Romulea, Crocus, Iris, Gladiolus, Sisyrinchium und Hermodactylus ausgewählt ist.

7. Produkt nach dem vorhergehenden Anspruch, **dadurch gekennzeichnet, daß** der Extrakt ein Extrakt von einem pflanzlichen Material ist, das von der Gattung Iris stammt.

8. Produkt nach dem vorhergehenden Anspruch, **dadurch gekennzeichnet, daß** der Extrakt ein Extrakt von einem pflanzlichen Material ist, das von *Iris pallida* stammt.

9. Topische, kosmetische oder dermatologische Zusammensetzung, die die wie in einem der vorhergehenden Ansprüche definierte Kombination und einen kosmetisch oder pharmazeutisch akzeptablen, geeigneten Träger enthält.

10. Zusammensetzung nach dem vorhergehenden Anspruch, **dadurch gekennzeichnet, daß** die Phenolderivate in einer Menge von 0,001 bis 10 Gew.-%, vorzugsweise 0,1 bis 5 Gew.-%, bezogen auf das Gesamtgewicht der Zusammensetzung, verwendet werden.

11. Zusammensetzung nach einem der Ansprüche 9 und 10, **dadurch gekennzeichnet, daß** der Extrakt mindestens einer Iridacea in einer Trockenmenge von 0,0001 bis 20 Gew.-%, bezogen auf das Gesamtgewicht der Zusammensetzung, bevorzugter 0,001 bis 10 Gew.-% und noch bevorzugter 0,001 bis 5 Gew.-% verwendet wird.

12. Verwendung des in einem der Ansprüche 1 bis 8 definierten Produkts zum Bleichen und/oder Depigmentieren der Haut oder ihrer Anhangsgebilde in einer kosmetischen Zusammensetzung oder für die Herstellung einer pharmazeutischen Zusammensetzung.

13. Kosmetisches Verfahren zum Bleichen oder Depigmentieren der Haut oder ihrer Anhangsgebilde, **dadurch gekennzeichnet, daß** auf die Haut oder ihre Anhangsgebilde eine wie in einem der Ansprüche 9 bis 11 definierte kosmetische Zusammensetzung aufgetragen wird.

## Claims

1. Combination product consisting of the combination of an extract of at least one Iridaceae and at least one phenol derivative of formula (I): 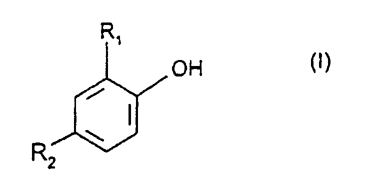 in which:
R₁ represents a hydrogen atom or an acid radical,
R₂ represents an atom or a radical chosen from the hydrogen atom, a radical of formula -NHCOCH₃ and a radical of formula -COR₃, in which R₃ represents a linear, branched or cyclized, saturated aliphatic chain, an unsaturated chain carrying one or more conjugated or nonconjugated double bonds, these chains containing from 2 to 22 carbon atoms and being capable of being substituted with at least one substituent chosen from halogen atoms, the trifluoromethyl group, hydroxyl groups in free form or in a form esterified with an acid having from 1 to 6 carbon atoms or alternatively with a carboxyl function, free or esterified with a lower alcohol having from 1 to 6 carbon atoms,
on the condition that when R₁ represents a hydrogen atom, R₂ does not represent a hydrogen atom,
and its cosmetically or pharmaceutically acceptable salts or esters.

2. Combination product according to Claim 1, **characterized in that** the phenol derivative is of formula (I) in which R₁ represents a hydrogen atom and R₂ represents the radical -NHCOCH₃.

3. Combination product according to Claim 1, **characterized in that** the phenol derivative is of formula (I) in which R₁ represents an acid radical, R₂ represents the radical of formula -COR₃ or a hydrogen atom.

4. Combination product according to Claim 1, **characterized in that** the phenol derivative is chosen from paracetamol, salicylic acid and the derivatives of salicylic acid.

5. Product according to Claim 4, **characterized in that** the salicylic acid derivatives are chosen from 5-(n-octanoyl)salicylic, 5-(n-decanoyl)salicylic and 5-(n-dodecanoyl)salicylic acids.

6. Product according to any one of the preceding claims, **characterized in that** the Iridaceae extract is obtained from an Iridaceae of a genus chosen from Romulea, Crocus, Iris, Gladiolus, Sisyrinchium or Hermodactylus.

7. Product according to the preceding claim, **characterized in that** the said extract is an extract of plant material obtained from Iris.

8. Product according to the preceding claim, **characterized in that** the said extract is an extract of plant material obtained from *Iris pallida.*

9. Cosmetic or dermatological topical composition comprising the combination as defined in any one of the preceding claims and a cosmetically or pharmaceutically acceptable appropriate vehicle.

10. Composition according to the preceding claim, **characterized in that** the phenol derivatives are used in a quantity of between 0.001 and 10%, preferably of between 0.1 and 5%, by weight relative to the total weight of the composition.

11. Composition according to either of Claims 9 and 10, **characterized in that** the extract of at least one Iridaceae is used in a dry quantity of between 0.0001 and 20% by weight relative to the total weight of the composition, more preferably of between 0.001 and 10% by weight, and still more preferably of between 0.001 and 5% by weight.

12. Use of the product defined according to any one of Claims 1 to 8 for lightening and/or depigmenting the skin or its superficial body growths in a cosmetic composition or for the manufacture of a pharmaceutical composition.

13. Cosmetic method for lightening or depigmenting the skin or its superficial body growths, **characterized in that** a cosmetic composition defined according to any one of Claims 9 to 11 is applied to the skin or its superficial body growths.
